# EUROPEAN PATENT APPLICATION

(11) **EP 2 674 031 A1**
(43) Date of publication of application: **18.12.2013**
(21) Application number: 13386018.9
(22) Date of filing: 24.05.2013
(51) Int. Cl.: A01N 25/06, A01N 33/12, A01N 31/02, A01P 1/00, A61L 2/22

(54) **Method for disinfecting ice making machines**

(30) Priority: 24.05.2012 GR 2012100274
(71) Applicant: Serafeimidis, Andreas, 13671 Axarnes (GR)
(72) Inventor: Serafeimidis, Andreas, 13671 Axarnes (GR)

(57) **Abstract**

The present invention provides a new method for the disinfection of ice making machines using aqueous disinfectant of Didecyldimethylammonium chloride with zero residual.

## Description

### DESCRIPTION OF THE INVENTION

Disinfection method of ice making machines using aqueous disinfectant with zero residual.

### BACKGROUND OF INVENTION

### Water for the ice production

According to the World Health Organization (WHO) water used for ice production must be free of solid particles, bacteria, taste and odor. Also, dissolved salts have to be at the lowest possible level.

Potable water is usually recovered from different sources, mainly groundwater and surface waters. Potable water used for food treatments as well as water used for ice production have to meet potable water standards, both in terms of health (absence of pathogens and toxic substances) and organoleptic point of view (taste, color, clarity). Potable water from private wells should be regularly disinfected and the effectiveness of the sanitization tested.

### Ice is a form of food

Usually, we overlook the fact that ice is also a form of food, consumed on daily bases as any other food. In this sense, talking about professional ice making machines, the implementation of safety rules is directly related to ensuring human health protection. Consequently, regardless of the type of ice makers or manufacturer, basic rules should be applied to prevent health risks.

### Ice contamination

Ice produced by ice making machines must meet the same quality standards as drinking water.

The contamination of the ice may be caused both by the water entering the machine, and the machine itself.

Contaminated ice is the dangerous source of different kind of pathogens and consequently one of the main causes of health problems. Foods contaminated by ice microbes deteriorate progressively and eventually may become dangerous to human health. Research findings confirm relationship between contaminated ice and intestinal diseases.

### Biofilm

Biofilm is build up when bacteria, associated with surfaces located in a damp environment, begin to secrete a sticky paste-like substance, which can be stuck on many different types of materials including metals, plastics and glass. Biofilm can be formed by one species of bacteria, but must often by different bacteria species like *Listeria, E.coli* and *Norovirus.*

Essentially, biofilm can be formed on any surface exposed to bacteria and small amount of water. Biofilm is the culprit that forms a protective layer for bacteria to thrive. Once bacteria grow into well-formed colonies, it is much more difficult to clean and disinfect. These slimy layers of biofilm must be removed and prevented from forming on surfaces of foodservice equipment. Biofilm can be quite difficult to remove from hidden surfaces, and can be an on-going source of disease and spoilage if not completely removed.

Ice making machines which are not properly cleaned and disinfected cerate an ideal environment for the increase of biofilm and bacteria that contaminate the ice.

### Risks scaling

Ice making unit and ice cubes storage unit may be the source of scaling and pollution, which may also contribute to microbial growth. Preventive descaling and disinfecting of ice machines are the best way to prevent the health risks.

### Identification of microbes

To evaluate the hygiene conditions of both food and ice, methods based on microbial identification and quantification are used. Identification and quantification levels of microbiological contamination considered to be a health risk are established by international and national laws and require the absence of coliforms (aerobic or optionally anaerobic gram-negative bacteria of the genus: *Klebsiella, Serratia, Arizona, Edwardsiella, Citrobacter* and *Providenza*), fecal origin coliforms of the genus : *Lactobacillus, Clostridium, Enterococcus, Escherichia coli, Enterobacfer, Klebsiella, Pseudomonas, Streptococcus* and *Candida.*

### Prevention from ice contamination

Our findings suggest that a serious problem exists in the field of public health safety caused by consumption of contaminated ice. This is a main reason for taking preventive protective action to protect the consumer by the application of a regular disinfection procedure of ice making equipment by certified disinfection service and mandatory HACCP application.

### Disinfection of water

Disinfection is usually the last step in the water treatment procedure (waste too) and aims the destruction or inactivation of pathogenic microorganisms in order to protect public health. All of decontamination methods are aimed at destroying bacteria, viruses and other microorganisms, which are likely to be carriers of disease.

Microbicidal activity of the disinfectant is achieved by destruction or weakening of the microorganism cellular structure organization, by disruption of the metabolism responsible for energy production, by disruption of biosynthesis and the microorganism growth.

Factors that influence the effectiveness of disinfection are the kind of disinfectant, the contact time and some of the water quality characteristics such as turbidity, presence of organic matter, pH, the temperature, the suspended substances etc.

The disinfectant should:
- Be effective in a wide antimicrobial spectrum,
- be low cost
- not create residual effects in the system and does not facilitate the uncontrolled growth of unwanted species,
- not create serious risks for personnel
- not create serious risk for personnel and the environment.

Note finally that pathogenic microorganisms have different resistance to various disinfectants. The seeds of bacteria and protozoa are most resilient, followed by viruses and bacteria.

### Classification of disinfectants

Water disinfectants may be divided into two major categories: non-chemical and chemical disinfectants.
- Non-chemical disinfectants are: the ultraviolet radiation (UV), radioactive irradiation, sterile filtration and heat.
- Chemical disinfectants are divided into oxidizing and non-oxidizing types.

**Oxidizing disinfectants** used in tanks of the water treatment facility to control the growth of microorganisms, discoloration, organoleptic characteristics (odor, taste), destruction of certain organic pollutants and metals precipitation are:
- Chlorine gas (Cl₂),
- Sodium hypochlorite (NaOCl) or sodium chlorite (NaOCl₂),
- Chlorine dioxide (ClO₂),
- Bromine (Br₂),
- Iodine (I₂),
- Hydrogen peroxide (H₂O₂),
- Ozone (O₃).

**Non-oxidizing disinfectants,** usually organic compounds that act independently on the pH value, have long life and are used to control microorganisms such as fungi, bacteria and algae:
- Methylene Bisthiocyanate,
- 2,2-dibromo-3-nitrilopropionamide,
- Chlorinated phenols,
- Isothiazolones,
- Bis(tributyltin) oxide.

### Chlorination

The most common disinfection method today is chlorination, which is achieved by adding either free chlorine or chlorine compounds to water. Below are the chemical formulas and the nomenclature of the different ions of chlorine and chloramines are associated with the chlorination.

| Chemical Formula | Nomenclature |
|---|---|
| Cl₂ | Chlorine |
| Cl⁻ | Chloride ion |
| ClO⁻ | Hypochlorite ion |
| ClO₂⁻ | Chlorite ion |
| ClO₃⁻ | Chlorate ion |
| ClO₄- | Perchlorate ion |
| Cl₂O | Chlorine monoxide |
| ClO₂ | Chlorine Dioxide |
| NH₂Cl | Monochloramine |
| NHCl₂ | Dichloramine |
| NCl₃ | Trichloramine |

Chlorine (Cl₂) is toxic for humans and animals. However, at low concentrations required for the destruction of pathogenic microorganisms chlorine is harmless. The usual concentration of chlorine in water does not exceed 1 mg / L (ppm), while even concentrations high as 50 mg / L have proved harmless to the human body. However, the chlorine taste and odor of drinking water, even at levels way below the dangerous, are quite unpleasant and make water undrinkable.

Chlorine as a strong oxidizing agent reacts readily with various compounds (Fe²⁺, NH⁴⁺, Mn²⁺, S²⁻, CN⁻, etc.) already present in water. The amount of chlorine needed for reaction with existing compounds, before starting the actual process of disinfection, is called required chlorine. Beyond its oxidative power, chlorine has also a great destructive potential towards the water-transmitted, pathogenic microorganisms. The amount of chlorine required for decontamination depends on the content of organic substances in water. In normal cases complete disinfection is achieved when, after mixing and oxidation, the concentration of free chlorine, which we refer below, is about 0,2 ppm. More chlorination gives to the water smell and taste while, on the other side, content below the above limit (0,2 ppm) does not ensure complete disinfection. To avoid adverse effect, the free chlorine content in the natural recipient must be checked first. The microbicidal capacity of the chlorine includes oxidation, degradation of the organic substances, hindering the development of algae and conversion of iron and magnesium to corresponding oxides. The action of chlorine is not instantaneous. The minimum time needed for chlorination is 15 -30 minutes in the best case. Generally, the rate of bacteria destruction depends on contact time with the chlorine. In chlorinated waters, the residual chlorine should be measured. The measured value shows if the chlorination was sufficient.

When the chlorine gas is added to water the hypochlorous acid (HOCI) is formed according to the equation:

Cl₂ + H₂O ↔ HOCI + H⁺ + Cl⁻

The hypochlorous acid is a weak acid which breaks into a hydrogen ion (H ⁺) and a hypochlorite ion (OCl⁻):

HOCl ↔ H⁺ + OCl⁻

The presence of hypochlorous acid in water depends mainly on pH value. Thus, at low pH values prevails HOCI, while at high values prevails the hypochlorite ion (OCl⁻).

The bactericidal activity of hypochlorous acid (HOCI) is stronger than the hypochlorite ion. The hydrochloric acid (HCl) produced during the reaction is neutralized by the bicarbonate ion (HCO3⁻) of water. Consequently, at higher pH values and lower temperatures the larger amount of chlorine is required for successful disinfection.

When used, sodium chlorite (NaClO₂) and chlorine gas, generate chlorine dioxide which is also the bactericidal agent:

2NaClO₂ + Cl₂ ↔ 2ClO₂ + 2NaCl

Chlorine dioxide is a gas that remains stable in water as a residue for a short time and it is highly active at high values of pH.

When sodium hypochlorite is used, hypochlorous acid (NaOCl) is produced which, at high pH values, converts into hypochlorite ion, whose bactericidal activity is not effective:

NaOCl + H₂O ↔ HOCI + NaOH

In this case, a correction of the pH value is needed, and pH should be reduced below 7.5. Exposure to sunlight, temperature and traces of heavy metals together will further reduce the quantity of active chlorine, due to conversion to chlorate and sodium chlorite.

The amount of the total existing active chlorine, in the form of hypochlorous acid (HOCI) and hypochlorite ion (OCl⁻), is known as free available chlorine or chlorine residual. Note that by the term active chlorine is meant the sum of all the chlorine compounds available in water which have disinfectant action after some time.

Below is given a percentage of hypochlorous acid as free available chlorine for different values of pH.

| pH | Percentage of HOCl as free Cl₂ |
|---|---|
| ≤ 6.7 | 95% |
| 7.0 | 80% |
| 8.0 | 30% |
| 9.0 | 5% |

For small plants, it is more economical to use calcium hypochlorite (Ca(OCl)₂) as a disinfectant instead of chlorine. This substance reacts with water and releases chlorine. The chlorination of water must be properly and systematically monitored in order to secure that only small amount of chlorine and disinfection by-products reach consumers.

With regard to the waste water disinfection using chlorine, it is emphasized the presence of chlorination by-products (such as trihalomethanes, THM). Chlorine reacts with organic compounds forming haloformate or trihalomethanes such as chloroform CHCl₃. All concerns about the potential effects of trihalomethanes on human health began with the hypothesis that the chloroform is carcinogenic. Today, however, research confirmed additional side effects such as infertility, kidneys and liver damage, effects on kidney or hematopoietic system etc.

### Ozonation

In recent years, different technologies have been developed for water and wastewater disinfection including the disinfection with ozone (O₃) which is considered to be particularly effective. Additionally, international experience has shown that the use of ozone is ideal for advanced treatment of wastewater, for recycling and reuse.

Ozone has a very strong and rapid disinfectant action which includes destroying viruses. The amount of ozone required for disinfection biologically treated wastewater is 15 - 20 g/m³ and the duration of the reaction is 15 to 30 minutes. For complete destruction of cellular links, even in the case of viruses, required residual concentrations varies from 0.2 to 0,5 mg/L with a contact time of 6 minutes.

The ozone can be generated from the air that has been suitably treated or from pure oxygen. The ozone gas may be produced by electrical discharges between two electrodes at 10 to 20 kV voltage. The reaction of ozone production takes place in a suitable reactor named ozonator and consists of a vertical or horizontal cylindrical container where a certain number of stainless steel tubing are bonded firmly to both ends of the container and adapted to a compact construction. On the outer side of the stainless steel tubes the cooling liquid (water) flows into the vessel in order to remove heat evolving during the ozone production. This method of construction prevents contact between cooling liquid and high voltage electrodes. Metal pipes are playing the role of pockets where specially calibrated glass tubes are inserted whose inner surface is a metal high voltage electrode. The high precision gap formed between the inner metal surface of the tubes and the outer glass surface, regulates the exact quantity of ozone generated. In each metallic case enters one glass tube. The high potential is applied between the metal and the metal surface of the glass tubes (high voltage electrodes), producing a silent electric discharge along the pipe. Under these conditions the produced ozone gas exits the vessel at the desired concentration. Ozone is produced in "spot", with unique requirements electricity and cooling water.

Ozone can be used for deodorizing the water. The organic compounds containing sulfur and nitrogen are the main causes of odors. Small quantities of ozone of the order of 1-2 mg/L added to water are capable of oxidizing these compounds. Additional benefit of the ozone deodorizing ability is that it prevents the recurrence of odors. Note that the gas flow consists of 95% of air providing aerobic conditions during the ozone treatment, which prevents the formation of odors. The action of ozone deodorization is based on both antimicrobial activity through oxidation of protein structures (bacteria or virus) and oxidation of odorous compounds, and formation of unstable compounds with the vectors of odors (urea, phenols etc.).

### Advantages of disinfection with ozone

- Lack of security problems related to transportation and storage
- Exquisite disinfectant properties. Rapidly destroys microorganisms resistant to chlorine or other disinfectants such as amoebae, beans, fungi, slime mold, algae, seeds and cysts
- Short application times (the ozonation requires about 10 min while the chlorination 30 - 35 min). The disinfection takes place almost instantaneously
- Less sensitivity to the pH and temperature. The existing disinfecting ability will be steady within pH 6 - 10 and temperature range 2 - 30 °C
- Improves the quality of treated water due to the high concentration of dissolved oxygen in the gas exit
- Increases total dissolved solids in the effluent
- Reduces coloration and turbidity of the waste
- Compared to chlorine residual, the residual ozone disinfection is an equally toxic process, however, it decomposes very quickly and it does not create problems in the receiving water.

### Disadvantages of disinfection with ozone

- High fixed and operational costs
- Certain industrial wastes contain contaminants that consume oxidant, which can make the ozonation ineffective
- Requires pilot installation to find the optimal dose of ozone
- Newly formed compounds may be hazardous to human health such as epoxides, bromine and brominated organic compounds.

### Disinfection with ultraviolet (UV) radiation

The method of water and wastewater disinfection with ultraviolet light is particularly effective in terms of destruction of unicellular microorganisms, such as pathogenic viruses. The destruction of microorganisms is the result of the radiation absorbed by the genetic material (DNA) of the cell. Maximum destructive ability of ultraviolet radiation is accomplished at a wavelength of 250 - 265 nm, indicating the maximum absorption of nucleic acids.

Although all microorganisms are susceptible to UV radiation, the sensitivity varies depending on the resistance to penetration of ultraviolet radiation. The chemical composition of the cell wall and its thickness determines the resistance of microorganisms to ultraviolet radiation. Additional treatment of affected molecules, after DNA exposure to ultraviolet radiation, using visible blue lights (310 - 500 nm) can result in the repair of damage and should be avoided. This phenomenon relates only to certain microorganisms and generally not viruses. For example, *Streptococcus* does not have the photo-repair ability while the *Shigella* has it.

Measure of the effectiveness of disinfection with ultraviolet radiation represents the amount of energy-dose absorbed by the microorganism. This amount is the product of the rate at which the energy is supplied (intensity) and the time interval during which the microorganism is exposed. Nevertheless, increasing the dose often results in reduced disinfectant action, due to the adsorption of bacteria in the suspended particulates. Another parameter to be taken into consideration is that certain organic and inorganic compounds present in water absorb the wavelength of the emitted ultraviolet radiation. The dose of radiation depends on the radiation intensity (energy) and the exposure time (duration of irradiation). It is inversely proportional to the irradiated surface.

### Advantages of UV disinfection

- It does not introduce chemical changes to the waste water so does not alter their impact on the receiving water;
- There is no toxic residual concentration;
- It is effective for many different kinds of microorganisms;
- The equipment takes up little space and is relatively inexpensive.

### Disadvantages of UV disinfection

- Lack of a measurable residual amount (difficulty relating to the direct control of the process);
- Lack of methods for measuring the dose, which also hampers the control;
- Probability of photo-repair of cells torn from UV radiation,
- Decreasing effectiveness of radiation and lifetime of UV-lamps;
- Lamps and reflective surfaces experience decay over time.

### Comparison of three methods of decontamination and disinfectant action

Briefly, follows a comparison of ozonation, disinfection with UV and chlorine-based disinfectant capacity, the hazards of the materials used and the economy.

| CHARACTERISTICS | DISINFECTION METHOD | | |
|---|---|---|---|
| | Ozonation | UV | Chlorination |
| Remove coliforms | Very good | Very good | Very good |
| Virus Removal | Very Good | Good | Fair |

| Microorganism regrowth chances | | | |
|---|---|---|---|
| | None | Minimum | Significant |
| Effect on recipient | None | None | Many solids |
| Disinfection byproducts | None | None | Haloformate |
| Risk of byproducts | Zero | Zero | Great |
| Hazard chemicals used | None | None | Great |
| Installation costs | Important | Important | Medium |

| Instrument operation and maintenance cost | | | |
|---|---|---|---|
| | Medium | Important | Medium |
| Extent required installation | Medium | Small | Large |

Ozone acts directly and destroys the cell membrane itself, and does not affect the pH significantly so does not require additives for the correction. If damaged, the cell membrane does not retain the cellular material which results in complete destruction of the cell. Cases where regrowth of microorganisms happened have not been reported. Finally, it has been established and proven effective action of ozone on species of microorganisms that survive chlorination and disinfection with UV. Ozone has a very short shelf life (30 min). It is characteristic that exposure to 1000 ppm of ozone for 30 sec may cause slight irritation while equivalent exposure to chlorine gas (Cl₂) or chlorine dioxide (ClO₂) is often fatal. The production and consumption of ozone is created in a closed circuit and excess destroyed in a thermo catalytic reactor (ozone destroyer). Not only that ozone does not create dangerous byproducts, but it also has a positive effect on the physical condition of the recipient. Cases where recipients were contaminated with toxic substances have not been observed, although these compounds can theoretically be formed, however, they are highly unstable and transform into inert organic and inorganic compounds before leaving the ozone tank-waste. Ozonation process undertakes simultaneous actions of color removal, deodorization, increase of dissolved oxygen in the hazardous waste and destruction of organic substances.

**Ultraviolet radiation,** essentially acts as "sterilant" to microorganisms, seeking to prevent their proliferation. The term "sterilization" of the microorganisms means degradation of DNA and RNA, preventing the transfer of genetic material and thus the reproduction of microorganisms. Nevertheless, several microorganisms are capable of repairing the damaged germ-plasma, reducing the disinfecting effect of ultraviolet radiation. A method of disinfection using ultraviolet radiation is facing serious problems caused by suspended solids. It has also been reported in the literature that the penetration of UV radiation into a particular layer of distilled water is reduced to a very low rate if, for example, ions of Iron are present in water sample. It is noticed a large decrease of the disinfectant action of UV radiation when dirt and grease accumulate on the surface of the lamps and reflectors.

During **chlorination,** the oxidation agent is hypochlorous acid (HOCI) which requires the diffusion through the cell membrane in order to destroy the cellular material. The chlorine penetrates the cell membrane and inactivates certain enzymes, as for example in bacteria, while regarding viruses and other microorganisms the mode of action has not been fully explained, and is likely to directly affect DNA and RNA in the nucleus. The process of inactivation of enzymes has been found to be reversible. The action of chlorine against viruses is less effective than that of ozone. Both the chlorine gas (Cl₂), and Chlorine dioxide (ClO₂) are toxic gases and any leakage accident may cause serious consequences. Simultaneously, the ClO₂ is highly unstable even in aqueous solutions, particularly if compressed. The disposals of chlorinated waste into surface waters or ground waters create a huge problem in the ecosystem and the consequences are even greater if those waters are used as water sources. The Cl₂ reacts with organic compounds forming haloformates (trihalomethanes, Chloroform CHCl₃, Bromodichloromethane CHCl₂Br, Chlorodibromomethane CHClBr₂ and Bromoform CHBr₃). Chloroform is carcinogenic, while the three brominated haloformates, unchecked for carcinogenicity, are mutagenic. The use of produced ClO₂ reduces the concentration of haloformate, but recent research has shown that this is likely to affect the hematopoietic system. To address these problems the method of dechlorination has been developed by adding SO₂ before the delivery to the final recipient. However, even small disturbances of added SO₂ can lead to depletion of dissolved oxygen, increased BOD₅ and reduced pH.

### Patents

US20070949577: describes an ice storage device with an ozone generator for disinfecting ice.

JP20060114195, JP20050325457: describe industrial refrigerator which includes a generator for UV disinfection ice.

US 20040816805: describes an icemaker comprising an ozone generator and a generator with ultraviolet radiation for decontamination of ice.

### DETAILED DESCRIPTION OF THE INVENTION

In the present invention, a new method for the disinfection of ice making machines has been presented, based on usage of aqueous disinfectant which contains Didecyldimethylammonium chloride aerosol with zero residual.

### Composition of aqueous disinfectant

Aqueous disinfectant containing as the main disinfectant active substance Didecyldimethylammonium chloride (DDAC)

| | |
|---|---|
| Didecyldimethylammonium chloride | 4 ppm - 12 ppm |
| Ethanol | 200 ppm - 600 ppm |
| Isopropanol | 1 ppm - 5 ppm |
| Methoxypropanol | 10 ppm - 30 ppm |
| Water | q.s. |

**Efficacy studies** demonstrating the effect of the product against microorganisms:

| | |
|---|---|
| *Staphylococcus aureus* | ATCC 6538 |
| *Enterococcus hirae* | ATCC 10541 |
| *Escherichia coli* | ATCC 10536 |
| *Pseudomonas aeruginosa* | ATCC 15442 |
| *Candida albicans* | ATCC 10231 |
| *Aspergillus niger* | ATCC 16404 |

Combats microorganisms: *Staphylococcus aureus, Enterococcus Hirae, E. coli, Pseudomonas aeruginosa, Listeria monocytogene, Salmonella, Candida albicans, molds, Fungi, Yeast.*

Safety assessment of all components prove that they are not toxic under normal application conditions. Basis of stability studies and the safety assessment of the product under normal conditions of use, the product is not dangerous to humans, animals and the environment.

### Required equipment for disinfection of ice making machines

Suction pump for liquid residual
Sprinkler for spraying of aqueous disinfectant

### Method for disinfection of the ice making machines

The invention of the method is characterized by the application of the disinfectant as small particles with a spray nozzle.The aqueous disinfectant is introduced into the ice maker machine using the air as a carrier, in the form of aerosol, particles consisting from air and aqueous disinfectant.

The aerosol is a dynamic system continuously changing due to the condensation of vapor, evaporation of liquid components of the particles, coagulation of small particles to large particles, and the deposition of particles in the surrounding walls. The aerosol behaves as a gas and diffuses throughout the icemaker. The particles of the disinfecting solution associate with pathogens. In this process, due to the interaction with the disinfectant, the pathogens are neutralized. Characteristics of aerosols, comparable to that of gases, depend mainly on the size of the particle diameter. The rate of particle sedimentation depends on the gravity force and air resistance. Since the gravitational force depends on the mass of the particles and the friction of the air, i.e. it depends on the cross section of the surface and velocity, a reduction of the particle diameter will reduce consequently the sedimentation rate by a factor 0.7. The result is that the smaller particle diameter will result with better dispersion of disinfection liquid through the space. The diameter of 200 microns is a critical limit. Below this diameter, particles are dispersed in a gas-like state. In contrast to conventional systems for cleaning and disinfection, the method presented has no plans to insert the nozzle of the sprayer in internal parts of the icemaker. Since the disinfectant is distributed in the corresponding space similar to a gas, a fuel inlet is sufficient to cover the entire space with the mixture and to disinfect the accessories within the space of the icemaker.

### Disinfection procedure includes the following steps:

1. Stop operating the icemaker for 30 minutes.
2. Clean the icemaker of the possible presence of the ice and water using the suction pump.
3. Chemical cleaning icemaker - desalination - optional cleaning.
4. Disinfection of the icemaker by spraying all disputed surfaces with the solution - aerosol of aqueous disinfectant.
5. Apply disinfection procedure during period not less than 2 to 5 minutes.
6. Rinse residues from the surfaces of the device by spraying pure water with the aid of the suction pump.
7. Finally, sampling of the last part of the residue and test for
   - Assay of any by chance residual disinfectant
   - Microbiological control.
8. Startup the icemaker.

### Advantages of the method of disinfection with aqueous disinfectant

1. Fast and safe application of the icemaker disinfection.
2. Guarantees 99.99% killing of pathogenic microorganisms
3. It is effective for many different species of microorganisms.
4. Zero residual.
5. Does not stop functioning ice making machines for over an hour
6. Consistent with the issues of HACCP.
7. Existence of a measurable residual amount - ease of the immediate control of the process.
8. Existence of methods for measuring dose of disinfectant, which helps control.

### Advantages of the active substance in the aqueous disinfectant

1. The active ingredient Didecyldimethylammonium chloride is fully biodegradable and does not result in chemical changes in waste so does not alter their impact on aqueous acceptor.
2. Excellent disinfectant properties - rapidly destroys microorganisms.
3. It is active to all classes of microorganisms that occur or are expected to occur in the system.
4. Small application times (requiring 2 to 5 minutes, while the ozonation takes about 10 min and the chlorination 30 - 35 min). The disinfection takes place almost instantaneously.
5. There is no possibility of remedying the damage of cells affected by the action of the disinfectant.
6. Does not cause side effects on the system and does not facilitate the uncontrolled growth of unwanted species.
7. There is no toxic residual concentration.
8. There is no development of compounds hazardous to health.
9. Anti odours action.
10. The activity of the disinfectant is not affected by water hardness.
11. It is not sensitive of pH and temperature.
12. Does not increase the total dissolved solids in the treated effluent.
13. It does not react with existing salts, thus it is not consumed and does not require larger quantity. Therefore, it decreases the costs which makes its use advantageous.
14. It is not harmful to personnel and the environment.
15. Removal of coliforms: Very good.
16. Virus Removal: Good.
17. Chances of regrowth of microorganisms: None.
18. Disinfection by-products: None.
19. Risk of residual byproducts: Zero.
20. Hazard of chemicals used: None

### Advantages of the required equipment for disinfection

1. The equipment is external and takes up little space.
2. It is economic - does not require expensive devices (UV lamp, ozonizers etc.)
3. Operation and maintenance cost is low.

### EXAMPLE

Preparation of an aqueous disinfectant with Didecyldimethylammonium chloride as active substance, 100 liters:

| | |
|---|---|
| Didecyldimethylammonium chloride | 0.85 g (8.5 ppm) |
| Ethanol | 40.00 g (400 ppm) |
| Isopropanol | 0.34 g (3.4 ppm) |
| Methoxypropanol | 2.00 g (20 ppm) |
| Purified water | q.s.100 L |

## Claims

1. A method of disinfecting of ice making machines **characterized by** the following features:
A. Use of aqueous disinfectant containing Dimethyldidecylammonium chloride in form of aerosol.
B. Quick action, 2 to 5 minutes disinfection time.
C. Safe and effective enforcement against pathogenic and non-microorganisms.
D. Zero residual.

2. An aqueous disinfectant composition according to claim 1 **characterized in that** it contains as the active substance Didecyldimethylammonium chloride. The composition is **characterized by** the following formulation:
| | |
|---|---|
| Didecyldimethylammonium chloride | 0.85 g (8.5 ppm) |
| Ethanol | 40.00 g (400 ppm) |
| Isopropanol | 0.34 g (3.4 ppm) |
| Methoxypropanol | 2.00 g (20 ppm) |
| Purified water | q.s.100 L |
